# EUROPEAN PATENT APPLICATION

(11) **EP 4 039 796 A1**
(43) Date of publication of application: **10.08.2022**
(21) Application number: 20870724.0
(22) Date of filing: 01.10.2020
(51) Int. Cl.: C12N 5/07, C12N 1/00, C12N 1/12

(54) **PRODUCTION METHOD FOR COMPOSITION FOR CELL CULTURING, COMPOSITION FOR CELL CULTURING OBTAINED BY SAME, AND CELL CULTURING METHOD USING SAME**

(30) Priority: 01.10.2019 JP 2019181336
(71) Applicant: Tokyo Women's Medical University, Tokyo 162-8666 (JP)
(72) Inventor: SHIMIZU, Tatsuya, Tokyo 162-8666 (JP); HARAGUCHI, Yuji, Tokyo 162-8666 (JP); ASAHI, Toru, Tokyo 169-8050 (JP); OKAMOTO, Yuta, Tokyo 169-8050 (JP)
(74) Representative: Ricker, Mathias
(86) International application number: PCT/JP2020/037471
(87) International publication number: WO 2021/066113

(57) **Abstract**

The present invention provides a production method for a composition for cell culturing. This production method comprises: (1) a step for subjecting algae to an acid hydrolysis treatment and/or an alkali hydrolysis treatment; (2) a step for neutralizing the hydrolysis product obtained in the step (1) to obtain an algae extract; and (3) a step for mixing the algae extract with a medium for cell culturing, wherein the medium for cell culturing does not substantially contain L-glutamine.

## Description

### FIELD

The present invention relates to a production method for a composition for cell culturing, to a composition for cell culturing obtained by it and to a cell culturing method using it.

### BACKGROUND

Mammalian cells are used in a diverse range of research and industrial fields including medicine, pharmacy, science and technology, agriculture and veterinary medicine. Such research has helped to elucidate the causes of diseases and has led to methods for their treatment. Biomedicines and vaccines produced from cells are used for treatment and prevention of a large host of diseases. Cultured cells have also contributed in a major way to development in basic academic fields such as cell biology, biochemistry, genetics, physiology and molecular biology. More recently, cultured muscle cells have been used as cell sources for "cultured meat". The protein production efficiency for "cultured meat" is much higher than by production via conventional livestock breeding, and based on data from published reports, since meat production has traditionally depended on livestock breeding, switching to cultured meat production systems can result in reduction of up to 99% for utilization of land, 96% for water consumption, 96% for greenhouse gas emissions and 45% for energy consumption. It is believed, therefore, that food production systems allowing continuous meat production using cultured cells will become more widely popular in the future. Using cultured mammalian cells as a food source will require very large amounts of cells.

Since culture media are essential for culturing of mammalian cells, mass quantities of culture media are used for cell research. Such culture media contain various types of nutrients which consist mainly of glucose and amino acids. The nutrients are generally derived from cereals or heterotrophic microorganisms, the latter also requiring nutrients derived from cereals. Chemical fertilizers and agricultural chemicals are necessary in large amounts for cereal cultivation, which in turn requires massive amounts of energy and can lead to generation of more greenhouse gases (GHG) and cause environmental pollution. Cereal production is in turn significantly influenced by environmental effects such as global warming and environmental pollution.

At the current time, over 150,000 species of algae have been registered in AlgaeBase (http://www.algaebase.org/), with the number continuing to be updated on a daily basis. Numerous different algae traits exist because of the huge variety of their species, ranging from species that are able to grow in environments of either purified water or seawater, or both, to basophilic and alkaliphilic species. A wide variety of uses are currently under development to take advantage of those traits. Such development includes drainage treatment utilizing the nitrogen assimilation properties of microalgae, and fixation of carbon dioxide gas generated by combustion of fossil fuels (NPLs 1 to 3).

Microalgae synthesize nutrients such as carbohydrates (saccharides) and lipids from carbon dioxide by photosynthesis, and produce amino acids using nitrogen gas/ammonia/nitrates. Microalgae also produce various nutrients by utilizing solar energy and inorganic materials. The nutrients produced by microalgae are currently being actively utilized in the fields of energy and food industry. For example, lipids extracted from microalgae are considered promising as an alternative energy source to petroleum (NPL 4). Glucose extracted from microalgae is also utilized as a nutrient for bioethanol-producing yeast (NPLs 5 to 6). Nutrients containing amino acids produced by microalgae are also used in nutritional supplements, pet foods and soil fertilizers (NPLs 7 to 10). It is generally thought that microalgae are the photosynthetic organisms that synthesize nutrients most efficiently without residue (NPL 11).

Culturing of cells *in vitro* often requires fetal calf, cow neonatal, horse, chicken or rabbit serum in addition to synthetic medium containing amino acids, vitamins and minerals. Serum contains factors necessary for cell growth, such as growth factors, which are often essential for growth of the cells. However, because serum is unstable and may differ in quality depending on the lot, while also being problematically expensive, there is a need to find alternative substances. In light of these problems, PTLs 1 and 2 describe microalgal extract that can partially substitute for the function of serum in cell culturing. However, these publications nowhere mention that cells can be cultured by mixing of an microalgal extract with synthetic media (such as inorganic salt medium) that lack components such as amino acids (especially glutamine) and vitamins that are essential for cell culturing.

### [CITATION LIST]

### [PATENT LITERATURE]

[PTL 1] Japanese Unexamined Patent Publication SHO No. 57-65179
[PTL 2] Japanese Unexamined Patent Publication SHO No. 57-206384

### [NON PATENT LITERATURE]

[NPL 1] L. Wang, M. Min, Y. Li, P. Chen, Y. Chen, et al., Cultivation of green algae Chlorella sp. in different wastewaters from municipal wastewater treatment plant. Appl. Biochem. Biotechnol. 162 (2010) 1174-1186.
[NPL 2] F. Rossi, E.J. Olguin, L. Diels, R. De Philippis, Microbial fixation of CO2 in water bodies and in drylands to combat climate change, soil loss and desertification. N. Biotechnol. 32 (2015) 109-120.
[NPL 3] J. Cheng, Y. Zhu, Z. Zhang, W. Yang, Modification and improvement of microalgae strains for strengthening CO2 fixation from coal-fired flue gas in power plants. Bioresour. Technol. (2019)doi: 10.1016/j.biortech.2019.121850.
[NPL 4] I. Ajjawi, J. Verruto, M. Aqui, L.B. Soriaga, J. Coppersmith, et al., Lipid production in Nannochloropsis gaditana is doubled by decreasing expression of a single transcriptional regulator. Nat. Biotechnol.35 (2017) 647-652.
[NPL 5] S.H. Ho, S.W. Huang, C.Y. Chen, T. Hasunuma, A. Kondo, et al., Bioethanol production using carbohydrate-rich microalgae biomass as feedstock. Bioresour. Technol. 135 (2013) 191-198.
[NPL 6] G.E. Lakatos, K. Ranglova, J. C. Manoel, T. Grivalsky, J. Kopecky, et al., Bioethanol production from microalgae polysaccharides. Folia Microbiol. (Praha). (2019)doi: 10.1007/s12223-019-00732-0.
[NPL 7] S. McCusker, P.R. Buff, Z. Yu, A.J Fascetti, Amino acid content of selected plant, algae and insect species: a search for alternative protein sources for use in pet foods. J. Nutr. Sci. 3 (2014)e39.
[NPL 8] G. Gutierrez-Salmean, L. Fabila-Castillo, G. Chamorro-Cevallos, Nutritional and toxicological aspects of Spirulina (Arthrospira). Nutr. Hosp. 32 (2015) 34-40.
[NPL 9] E.T. Alori, O.O. Babalola, Microbial inoculants for improving crop quality and human health in Africa. Front. Microbiol. 9 (2018) 2213.
[NPL 10] M.I. Khan, J. H. Shin, J. D. Kim, The promising future of microalgae: current status, challenges and optimization of a sustainable and renewable industry for biofuels, feed and other products. Microb. Cell Fact. 17 (2018) 36.
[NPL 11] A.K. Singh, M.P. Singh, Importance of algae as a potential source of biofuel. Cell Mol. Biol. (Noisy-le-grand). 60 (2014) 106-109.

### SUMMARY

### [TECHNICAL PROBLEM]

It is an object of the present invention to establish a novel cell culturing system that lowers the risk of environmental load or environmental change.

### [SOLUTION TO PROBLEM]

The present inventors have conducted research and development based on examination of the aforementioned problem from many angles, in order to solve the problems described above. As a result it was found, surprisingly, that cells can be cultured in medium (for example, medium free of amino acids and/or vitamins) comprising added nutrients extracted from microalgae. Specifically, the invention encompasses the following.
[1] A composition for cell culturing comprising algae-derived components, wherein the components are obtained by the following steps:
   (1) subjecting algae to treatment by acid hydrolysis and/or alkali hydrolysis; and
   (2) neutralizing the hydrolysate obtained by step (1).
[2] The composition according to [1] above, wherein the cells are animal cells.
[3] The composition according to [1] or [2], wherein the algae are unicellular algae.
[4] The composition according to any one of [1] to [3] above, wherein the algae are at least one type selected from the group consisting of unicellular green algae, unicellular blue-green algae, unicellular red algae, unicellular charales and unicellular ulvophytes.
[5] The composition according to any one of [1] to [4] above, wherein the algae are at least one type selected from the group consisting of *Chlorococcum littorale*, *Stichococcus sp*., *Chlorella vulgaris* Beijerinck, *Euglena gracilis, Spirulina subsalsa* and *Arthrospira platensis.*
[6] The composition according to any one of [1] to [5] above, wherein step (1) is carried out under pressure.
[7] The composition according to any one of [1] to [6] above, wherein step (1) is carried out at 60°C to 200°C.
[8] A method of culturing cells in medium containing an added composition for cell culturing comprising algae-derived components, wherein the components are obtained by the following steps:
   (1) subjecting algae to treatment by acid hydrolysis and/or alkali hydrolysis, and
   (2) neutralizing the hydrolysate obtained by step (1).
[9] The method according to [8] above, wherein the cells are animal cells.
[10] The method according to [8] or [9] above, wherein the algae are unicellular algae.
[11] The method according to any one of [8] to [10] above, wherein the algae are at least one type selected from the group consisting of unicellular green algae, unicellular blue-green algae, unicellular red algae, unicellular charales and unicellular ulvophytes.
[12] The composition according to any one of [8] to [11] above, wherein the algae are at least one type selected from the group consisting of *Chlorococcum littorale*, *Stichococcus sp*., *Chlorella vulgaris* Beijerinck, *Euglena gracilis, Spirulina subsalsa* and *Arthrospiraplatensis.*
[13] The method according to any one of [8] to [12] above, wherein step (1) is carried out under pressure.
[14] The method according to any one of [8] to [13] above, wherein step (1) is carried out at 60°C to 200°C.
[15] A production method for a composition for cell culturing, wherein the method comprises the following steps:
   (1) subjecting algae to acid hydrolysis and/or alkali hydrolysis;
   (2) neutralizing the hydrolysate obtained from step (1) to obtain an algal extract; and
   (3) mixing the algal extract with cell culturing medium, wherein the cell culturing medium contains substantially no L-glutamine.
[16] The method according to [15] above, wherein the cell culturing medium is also substantially free of one or more amino acids selected from the group consisting of L-arginine, L-cysteine, glycine, L-histidine, L-isoleucine, L-leucine, L-lysine, L-methionine, L-phenylalanine, L-serine, L-threonine, L-tryptophan, L-tyrosine and L-valine.
[17] The method according to [15] or [16] above wherein the cell culturing medium contains substantially no vitamins.
[18] The method according to any one of [15] to [17] above, wherein the cell culturing medium contains substantially no glucose.
[19] The method according to any one of [15] to [18] above, wherein the cell culturing medium is an inorganic salt medium.
[20] The method according to any one of [15] to [19] above, wherein the cultured cells are animal cells.
[21] The method according to any one of [15] to [20] above, wherein the algae are unicellular algae.
[22] The method according to any one of [15] to [21] above, wherein the algae are at least one type selected from the group consisting of unicellular green algae, unicellular blue-green algae, unicellular red algae, unicellular charales and unicellular ulvophytes.
[23] The composition according to any one of [15] to [22] above, wherein the algae are at least one type selected from the group consisting of *Chlorococcum littorale*, *Stichococcus sp*., *Chlorella vulgaris* Beijerinck, *Euglena gracilis, Spirulina subsalsa* and *Arthrospiraplatensis.*
[24] The method according to any one of [15] to [23] above, wherein step (1) is carried out under pressure.
[25] The method according to any one of [15] to [24] above, wherein step (1) is carried out at 60°C to 200°C.
[26] A composition for cell culturing which is obtained by the method according to any one of [15] to [25] above.
[27] A cell culturing method using a composition for cell culturing which is obtained by the method according to any one of [15] to [25] above.

### [ADVANTAGEOUS EFFECTS OF INVENTION]

According to the method of the invention, nutrients necessary for cell culturing can be easily and economically extracted from microalgae, and it is thereby possible to provide a novel cell culturing system that reduces environmental load.

### BRIEF DESCRIPTION OF DRAWINGS

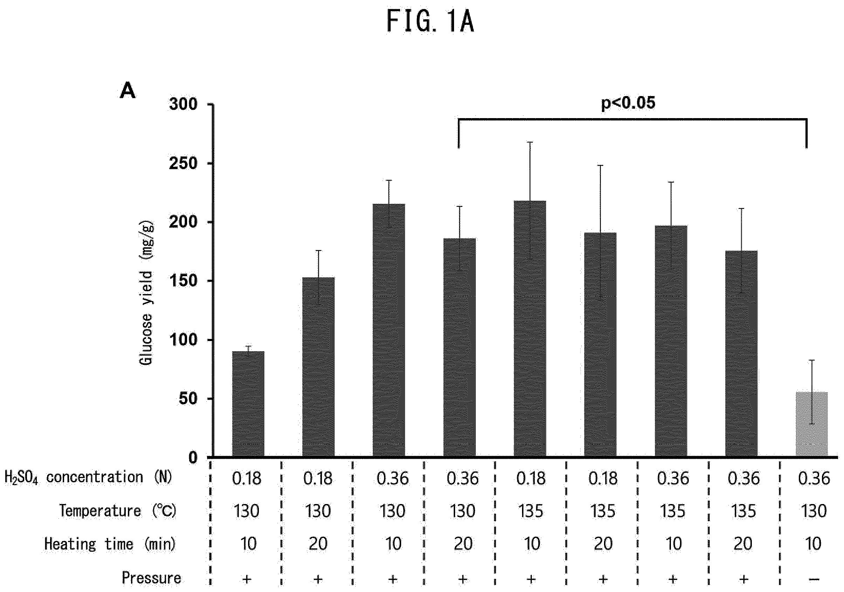

Fig. 1A shows glucose extraction from microalgae. (A) Glucose was efficiently extracted from the green alga *Chlorococcum littorale* under autoclave conditions. The data shown are mean ±SD (n = 3 to 6).

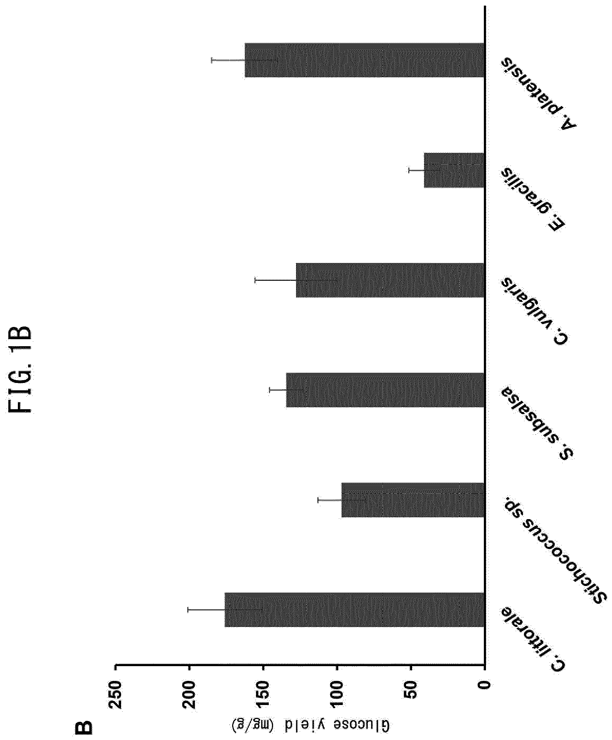

Fig. 1B shows glucose extraction from microalgae. (B) Glucose was more efficiently extracted from two microalgae (*Chlorococcum littorale* and *Arthrospira platensis*) among 6 different microalgae species (sulfuric acid normality: 0.36 N; reaction temperature: 130°C, reaction time: 20 min). The data shown are mean ±SD (n = 3 to 6). DMEM: Dulbecco's Modified Eagle Medium supplemented with 10% fetal bovine serum and 1% penicillin/streptomycin.

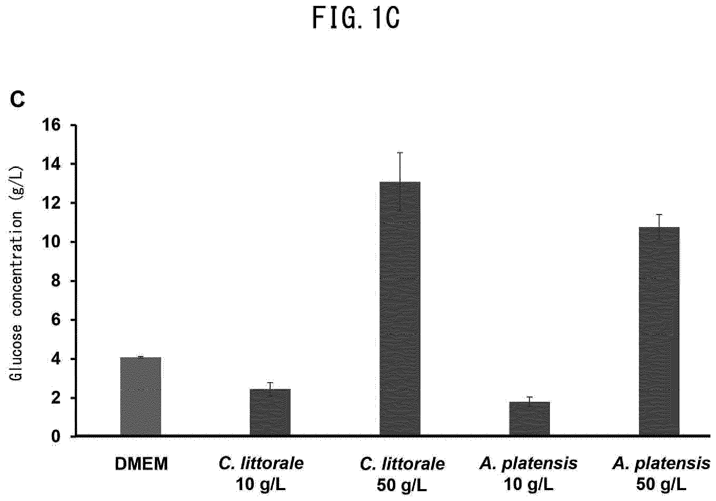

Fig. 1C shows glucose extraction from microalgae. (C) A high glucose yield was obtained in a manner dependent on the initial algae concentration (sulfuric acid normality: 0.36 N; reaction temperature: 130°C, reaction time: 20 min). The data shown are mean ±SD (n = 3 to 6). DMEM: Dulbecco's Modified Eagle Medium supplemented with 10% fetal bovine serum and 1% penicillin/streptomycin.

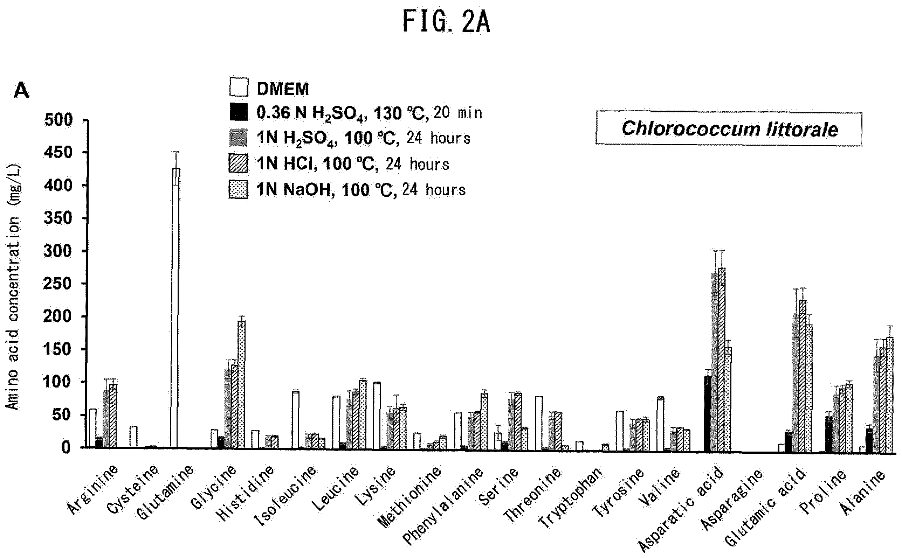

Fig. 2A shows amino acid extraction from microalgae. (A) Most of the amino acids were most efficiently extracted from *Chlorococcum littorale* by treatment with IN hydrochloric acid at 100°C for 24 hours. The data shown are mean ±SD (n = 3 to 7). DMEM: Dulbecco's Modified Eagle Medium supplemented with 10% fetal bovine serum and 1% penicillin/streptomycin.

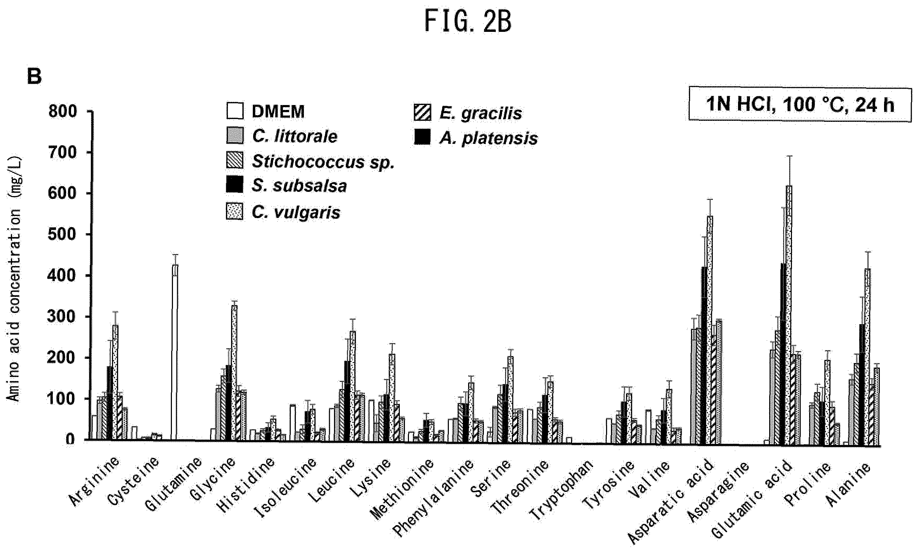

Fig. 2B shows amino acid extraction from microalgae. (B) Most of the amino acids were most efficiently extracted from *Chlorella vulgaris* Beijerinsk among the 6 microalgae species (hydrochloric acid normality: 1 N; reaction temperature: 100°C; reaction time: 24 hours). The data shown are mean ±SD (n = 3 to 4). DMEM: Dulbecco's Modified Eagle Medium supplemented with 10% fetal bovine serum and 1% penicillin/streptomycin.

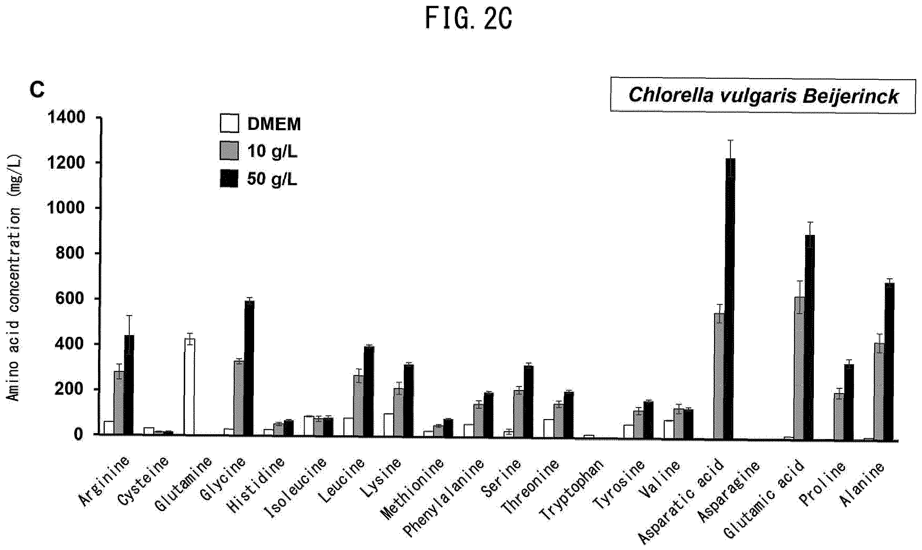

Fig. 2C shows amino acid extraction from microalgae. (C) A high amino acid yield was obtained in a manner dependent on the initial algae concentration (hydrochloric acid normality: 1 N; reaction temperature: 100°C, reaction time: 24 hours). The data shown are mean ±SD (n = 3 to 4). DMEM: Dulbecco's Modified Eagle Medium supplemented with 10% fetal bovine serum and 1% penicillin/streptomycin.

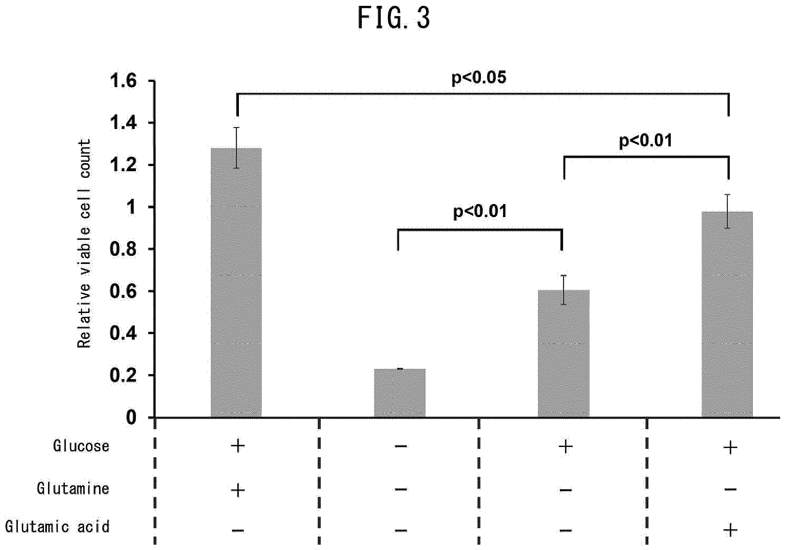

Fig. 3 shows the effect of glutamine and glutamic acid on mammalian cell culturing. C2C12 mouse myoblasts were cultured with mammalian cell medium containing glucose (final concentration: 25 mM)/glutamine (final concentration: 4 mM), or with glucose/glutamine-free medium with or without addition of glucose (final concentration: 25 mM) or glucose/glutamic acid (final concentration: 4 mM). The data are represented as mean ±SD (n = 7) for the relative viable cell counts calculated by absorbance.

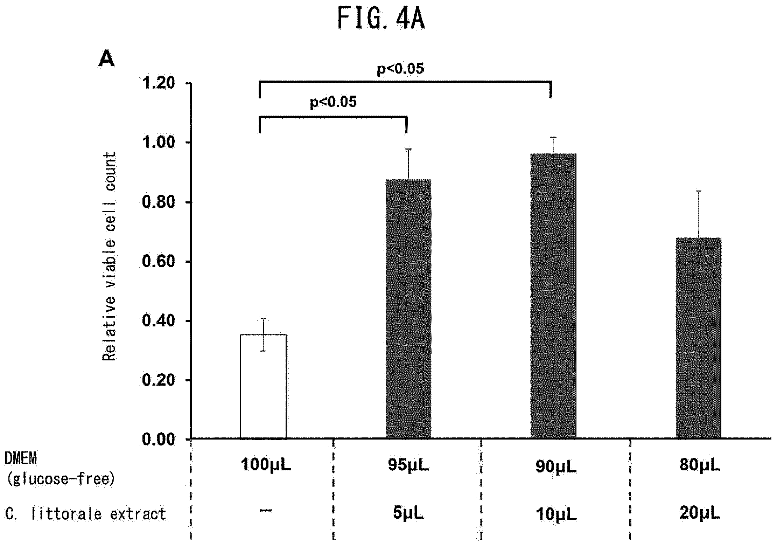

Fig. 4A shows culturing of mammalian cells using nutrient-deficient medium and algal extract. (A) C2C12 mouse myoblasts were cultured in glucose-free mammalian cell medium with or without addition of extract from *Chlorococcum littorale* (*C. littorale*). The data are represented as mean ±SD (n = 3 to 12) for the relative viable cell counts calculated by absorbance.

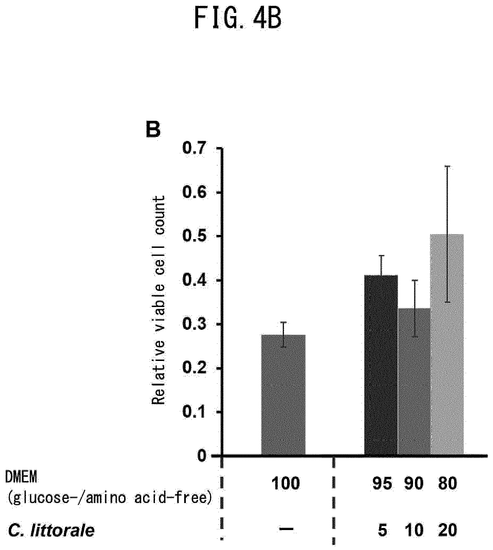

Fig. 4B shows culturing of mammalian cells using nutrient-deficient medium and algal extract. (B) C2C12 mouse myoblasts were cultured in glucose/amino acid-free mammalian cell medium with or without addition of extract from *Chlorococcum littorale* (C. *littorale*). The data are represented as mean ±SD (n = 3 to 12) for the relative viable cell counts calculated by absorbance.

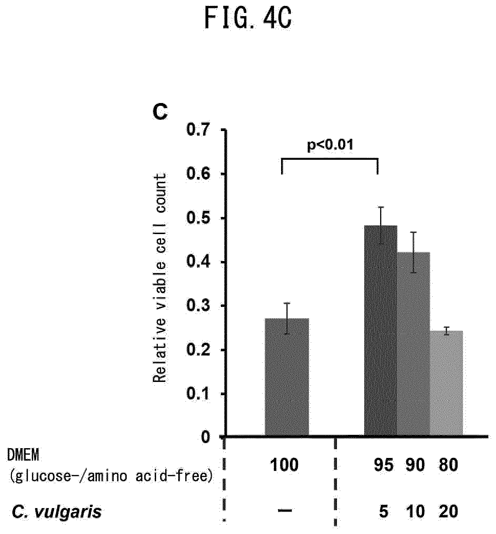

Fig. 4C shows culturing of mammalian cells using nutrient-deficient medium and algal extract. (C) C2C12 mouse myoblasts were cultured in glucose/amino acid-free mammalian cell medium with or without addition of extract from *Chlorella vulgaris* (*C. vulgaris*). The data are represented as mean ±SD (n = 3 to 12) for the relative viable cell counts calculated by absorbance.

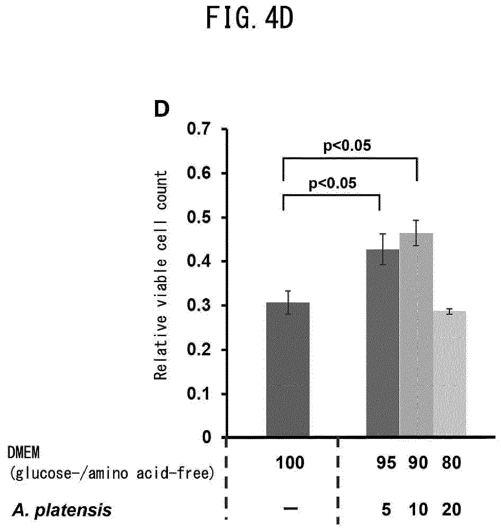

Fig. 4D shows culturing of mammalian cells using nutrient-deficient medium and algal extract. (D) C2C12 mouse myoblasts were cultured in glucose/amino acid-free mammalian cell medium with or without addition of extract from *Arthrospiraplatensis* (*A. platensis*). The data are represented as mean ±SD (n = 3 to 12) for the relative viable cell counts calculated by absorbance.

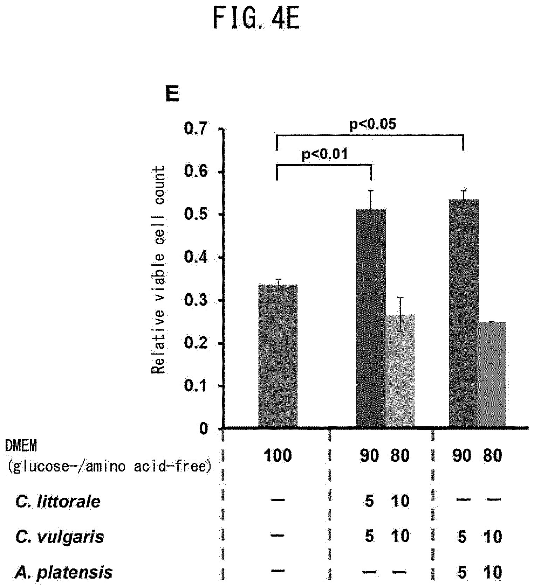

Fig. 4E shows culturing of mammalian cells using nutrient-deficient medium and algal extract. (E) C2C12 mouse myoblasts were cultured in glucose/amino acid-free mammalian cell medium with or without addition of extract from *Chlorococcum littorale* (*C. littorale*), *Chlorella vulgaris* (*C. vulgaris*) or *Arthrospira platensis* (*A. platensis*). The data are represented as mean ±SD (n = 3 to 12) for the relative viable cell counts calculated by absorbance.

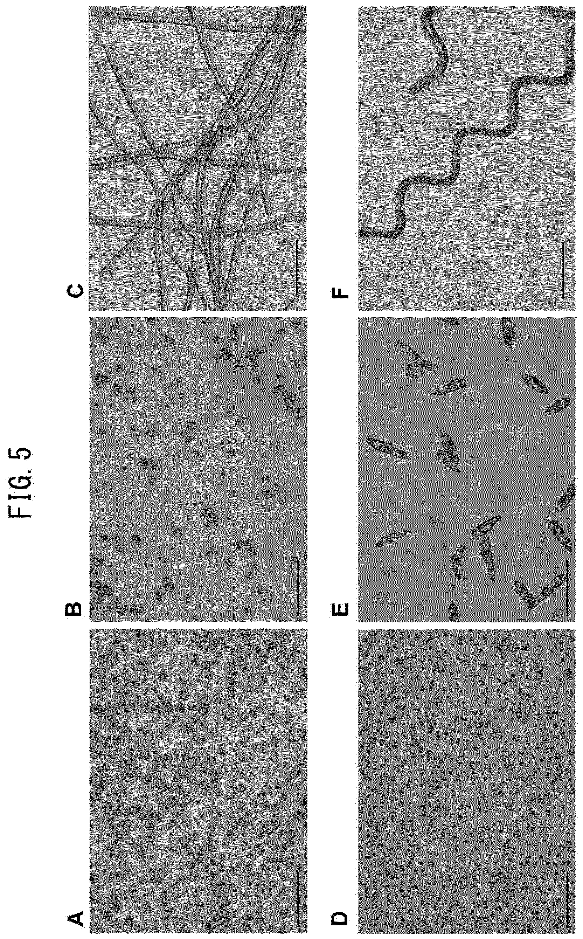

Fig. 5 shows photographs of the microalgae used in the Examples. (A) green algae *Chlorococcum littorale,* (B) *Stichococcus sp.,* (C) *Chlorella vulgaris* Beijerinck, (D) *Euglena gracilis*, (E) *Spirulina subsalsa* and (F) *Arthrospira platensis.* Scale: 50 µm.

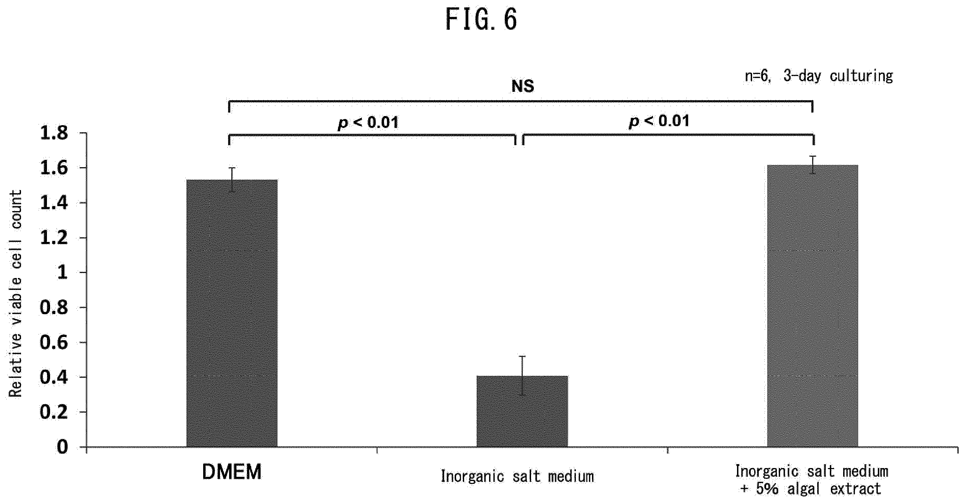

Fig. 6 shows culturing of mammalian cells using inorganic salt medium and algal extract. Cow muscle tissue cells were cultured in DMEM, or in inorganic salt medium with or without addition of extract from *Chlorella vulgaris* (C. *vulgaris).* The data are represented as mean ±SD (n = 6) for the relative viable cell counts calculated by absorbance.

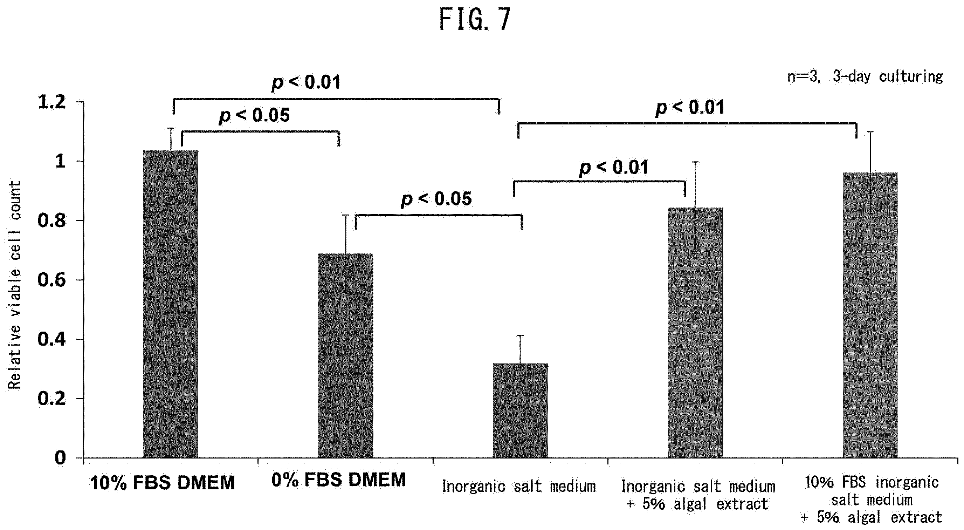

Fig. 7 shows culturing of mammalian cells using inorganic salt medium and algal extract. Cow muscle tissue cells were cultured in DMEM with or without addition of 10% fetal bovine serum (FBS), or in inorganic salt medium with or without addition of 10% fetal bovine serum (FBS) or extract from *Chlorella vulgaris* (C. *vulgaris).* The data are represented as mean ±SD (n = 3) for the relative viable cell counts calculated by absorbance.

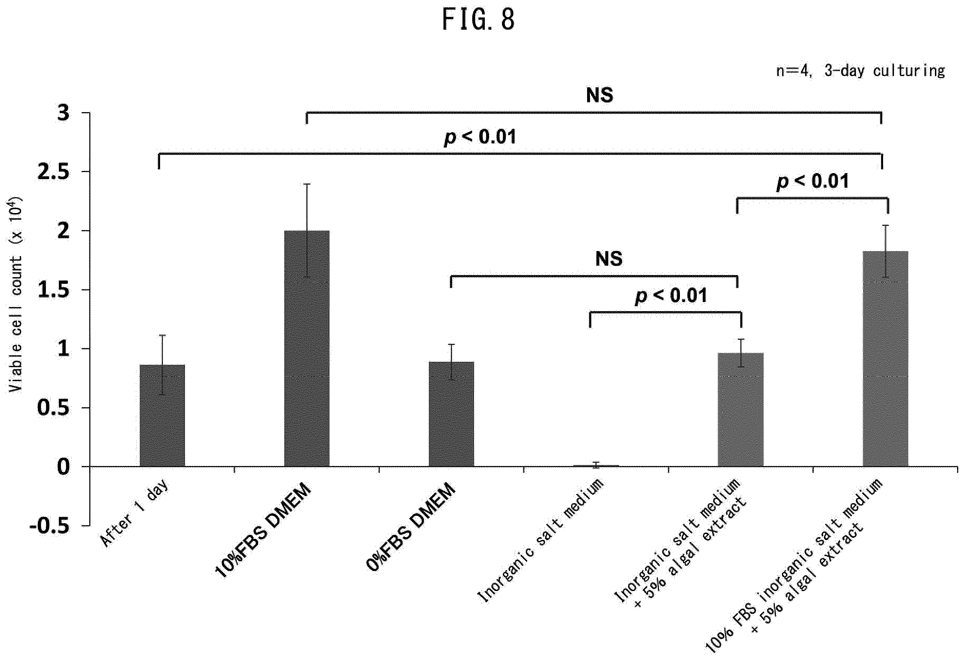

Fig. 8 shows culturing of mammalian cells using inorganic salt medium and algal extract. Cow muscle tissue cells were cultured in DMEM with or without addition of 10% fetal bovine serum (FBS), or in inorganic salt medium with or without addition of 10% fetal bovine serum (FBS) or extract from *Chlorella vulgaris* (C. *vulgaris).* The data are represented as mean ±SD (n = 4) for the relative viable cell counts.

### DESCRIPTION OF EMBODIMENTS

Embodiments of the invention will now be explained with reference to the accompanying drawings as necessary. The embodiments serve merely for example and the construction of the invention is not limited by the concrete constructions of the embodiments.

According to one embodiment, the invention provides a composition for cell culturing comprising an alga-derived component. The alga-derived component in the composition of the invention is obtained by the following steps:
(1) subjecting algae to treatment by acid hydrolysis and/or alkali hydrolysis, and
(2) neutralizing the hydrolysate obtained by step (1).

According to another embodiment, the invention provides a production method for a composition for cell culturing, wherein the method comprises the following steps:
(1) subjecting algae to acid hydrolysis and/or alkali hydrolysis;
(2) neutralizing the hydrolysate obtained from step (1) to obtain an algal extract; and
(3) mixing the algal extract with cell culturing medium, wherein the cell culturing medium contains substantially no L-glutamine.

According to yet another embodiment, the cell culturing medium to be used for the invention may be cell culturing medium that is also substantially free of one or more amino acids selected from the group consisting of L-arginine, L-cysteine, glycine, L-histidine, L-isoleucine, L-leucine, L-lysine, L-methionine, L-phenylalanine, L-serine, L-threonine, L-tryptophan, L-tyrosine and L-valine.

According to yet another embodiment, the cell culturing medium to be used for the invention may be cell culturing medium that contains substantially no vitamins. Cell culturing medium that contains substantially no vitamins to be used for the invention may be medium that is substantially free of one or more vitamins selected from the group consisting of pantothenic acid, choline chloride, folic acid, i-inositol, niacinamide, pyridoxine, riboflavin, thiamine, and pharmaceutically acceptable salts of the same.

According to yet another embodiment, the cell culturing medium to be used for the invention may be cell culturing medium that contains substantially no glucose, or it may be cell culturing medium that also contains substantially no pyruvic acid.

As used herein, "contain(s) substantially no (or substantially free of)" means containing substantially none of the substance in question, but it does not mean containing absolutely none of the substance, and for example, glucose may be present at less than 10 mg/L as the minimum limit of determination, amino acids (such as L-glutamine) may be present at less than 0.1 mg/L as the minimum limit of determination, or vitamins may be present at less than 1 µg/L as the minimum limit of determination.

Also as used herein, "algae" is a general term referring to organisms that produce oxygen by photosynthesis, with the exclusion of primarily land-inhabiting bryophytes, pteridophytes and spermatophytes. If given an environment necessary for photosynthesis, algae can produce oxygen and nutrients (such as glucose and amino acids) by themselves and proliferate. The present invention has been completed based on the finding that cells can be cultured in medium containing an added composition that comprises components extracted from algae.

The algae used for one embodiment of the invention may be "unicellular algae" (also known as "microalgae"). As used herein, "unicellular algae" refers to algae with individuals consisting of single cells, and it includes unicellular algae in which multiple unicellular algae cluster together to form groups. Examples are unicellular green algae in which chlorophyll a and b are the major pigments of the chloroplasts, unicellular blue-green algae (cyanobacteria) in which chlorophyll d is the major pigment, and unicellular red algae in which chlorophyll a and phycobilin proteins are the major pigments. More specific examples include the green algae *Chlamydomonas reinhardtii* of the class Chlorophyceae, order Chlamydomonadales, *Dunaliella salina* of the order Dunaliellales, *Volvox carteri* of the order Volvocales, *Chlorococcum littorale* of the order Chlorococcales, *Hydrodictyon reticulatum*, *Pediastrum duplex* and *Scenedesmus dimorphus* of the order Sphaeropleales, *Chlorella vulgaris* Beijerinck (chlorella) of the class Trebouxiophyceae, order Chlorellales, *Euglena gracilis* and *Euglena proxima* of the phylum Euglenozoa, class Euglenophyceae, order Euglena. Unicellular blue-green algae include *Acaryochloris marina*, *Spirulina subsalsa* and *Arthrospira platensis* of the phylum Cyanobacteria. Unicellular red algae include *Cyanidium caldarium* of the phylum Rhodophyta, class Cyanidiophyceae, order Cyanidiales, and *Galdieria partita* of the phylum Rhodophyta, class Cyanidiophyceae, order Cyanidiales. Unicellular charales include *Stichococcus sp.* of the phylum Chlorophyta, class Chlorophyceae, order Klebsormidiales. Unicellular ulvophyte algae include filamentous ulvophytes. The algae used for the invention may also be gene recombinant forms produced by genetic engineering of the aforementioned algae, and are not limited to the algae mentioned above.

The algae used for the invention may be natural algae or algae grown by publicly known culturing methods.

The algae-derived components in the composition used for the invention can be extracted by subjecting algae to acid hydrolysis and/or alkali hydrolysis treatment.

Biopolymers such as proteins and polysaccharides usually hydrolyze by mixing with strong organic or inorganic acids (such as hydrochloric acid, sulfuric acid, trifluoroacetic acid, p-toluenesulfonic acid or methanesulfonic acid), or strong alkalis (such as sodium hydroxide or potassium hydroxide) with addition of water, while heating (at 60°C to 200°C, preferably 80°C to 180°C and more preferably 90°C to 150°C, for example), producing oligomers or monomers (peptides and amino acids in the case of proteins, or oligosaccharides and monosaccharides in the case of polysaccharides). Therefore, the term "acid hydrolysis" as used herein refers to hydrolysis using a strong acid, and the term "alkali hydrolysis" refers to hydrolysis with a strong alkali. Publicly known "acid hydrolysis" and/or "alkali hydrolysis" may be used to obtain the composition of the invention. The reaction time for "acid hydrolysis" or "alkali hydrolysis" may be adjusted depending on the conditions such as the type, amount and concentration of algae or the pH and temperature of the reaction mixture, and it may be 1 to 1440 minutes, 3 to 720 minutes, 5 to 360 minutes, 5 to 120 minutes or 5 to 30 minutes, for example.

According to one embodiment, acid hydrolysis alone or alkali hydrolysis alone may be carried out to obtain a composition comprising algae-derived components, or both acid hydrolysis and alkali hydrolysis may be carried out. When both acid hydrolysis and alkali hydrolysis are carried out, the alkali hydrolysis may be carried out after acid hydrolysis or the acid hydrolysis may be carried out after alkali hydrolysis. Neutralizing treatment may also be carried out between the acid hydrolysis and alkali hydrolysis.

According to one embodiment, the algae used in step (1) may be subjected for drying treatment before acid hydrolysis and/or alkali hydrolysis.

According to another embodiment, step (1) may be carried out under pressure. The term "under pressure" as used herein may be air pressure conditions higher than atmospheric pressure, i.e., 1 atmosphere, such as 1.1 atmosphere or higher, 1.5 atmosphere or higher, 1.8 atmosphere or higher or 2 atmosphere or higher. For example, it may be 1.1 to 300 atmospheres, 1.5 to 200 atmospheres, 1.8 to 100 atmospheres, 2 to 50 atmospheres or 2 to 20 atmospheres. The pressure conditions may be conditions created using any apparatus or method, such as pressurized conditions produced with an autoclave.

The composition of the invention can be obtained by neutralizing the hydrolysate obtained from step (1) (neutralization step (2)). This allows the hydrolysate to be neutralized and used for culturing of cells. When acid hydrolysis has been carried out at the end of step (1), for example, a basic substance or its aqueous solution (sodium hydroxide, potassium hydroxide or an aqueous solution of the same) may be added for neutralization. When alkali hydrolysis has been carried out at the end of step (1), an acidic substance (such as sodium hydroxide or potassium hydroxide) or its aqueous solution (such as hydrochloric acid, sulfuric acid, trifluoroacetic acid, *p*-toluenesulfonic acid or methanesulfonic acid) may be added for neutralization.

The composition of the invention can be used for culturing of cells, such as prokaryotic cells (such as *E. coli*, lactic acid bacteria, *Bacillus subtilis* or cyanobacteria) or eukaryotic cells (such as yeast cells, plant cells, insect cells or animal cells), and especially animal cells. According to one embodiment, the source of animal cells to be cultured according to the invention may be a mammal, bird, amphibian, reptile or fish, and the source of mammalian animal cells may be a mouse, rat, rabbit, human, horse, cow, monkey, pig, dog, sheep, cat or goat, though with no limitation to these. The cells may be primary cells harvested from biological tissue, or from an established cell line, or pluripotent stem cells (such as ES cells, ntES cells, Muse cells or iPS cells), or tissue stem cells (such as mesenchymal stem cells), or cells obtained by inducing differentiation from such cells.

The composition used for the invention may comprise one or more algae-derived components, such as two or more algae-derived components, for example. For example, the composition may comprise a combination of a glucose-rich algae-derived component and an proteinogenic amino acid-rich algae-derived component.

The invention provides a method of culturing cells in medium containing an added composition for cell culturing comprising algae-derived components, wherein the components are obtained by the following steps:
(1) subjecting algae to treatment by acid hydrolysis and/or alkali hydrolysis, and
(2) neutralizing the hydrolysate obtained by step (1).

According to another embodiment, the method of culturing cells of the invention may also include (3) mixing the algal extract with cell culturing medium, wherein the cell culturing medium contains substantially no L-glutamine.

In the method of culturing cells of the invention, the medium to which the composition comprising the algae-derived component is added may be selected as appropriate for the type of cells to be cultured, but preferably the medium has buffering action, and it may be phosphate buffer solution, Eagle medium, Dulbecco's Modified Eagle Medium (DMEM), DMEM:F12 medium, Glasgow Minimum Essential Medium, Grace's Insect Medium, Ham's Medium, Iscove's Modified Eagle Medium, RPMI-1640 medium, L-15 medium or McCoy's 5A Medium, for example, which may also contains substantially no glucose and/or proteinogenic amino acids (one or more amino acids selected from the group consisting of such as L-glutamine, L-arginine, L-cysteine, glycine, L-histidine, L-isoleucine, L-leucine, L-lysine, L-methionine, L-phenylalanine, L-serine, L-threonine, L-tryptophan, L-tyrosine and L-valine). The culture medium may be liquid medium or solid medium. Addition of a composition comprising algae-derived components according to the invention to medium allows nutrients necessary for cell survival (such as glucose, proteinogenic amino acids and vitamins) to be supplied for cell culturing. The proportion in which the composition comprising algae-derived components is added to the medium may be adjusted as appropriate for the type of cells to be cultured, and the composition comprising the algae-derived components may be present at 0.1 to 99% (v/v), 1 to 80% (v/v), 2 to 50% (v/v), 3 to 20% (v/v), or 3 to 15% or 3 to 12% (v/v), for example, with respect to the total volume of the medium to which the composition comprising the algae-derived components is added.

### EXAMPLES

The present invention will now be explained in greater detail by examples, with the understanding that the invention is not limited in any way by the examples.

### <Example 1>

### 1. Materials and Methods

### 1-1. Microalgae culturing

For this experiment there were used four eukaryotic microalgae, the green algae *Chlorococcum littorale* (*C. littorale*) (NBRC 102761), *Stichococcus sp.* (NBRC 102709), (National Institute of Technology and Evaluation (NITE), independent chemical corporation, Chiba Prefecture, Japan), *Chlorella vulgaris* Beijerinck (C. *vulgaris)* (NIES-2170) and *Euglena gracilis* (*E. gracilis*) (NIES-49), and two prokaryotic microalgae, *Spirulina subsalsa* (*S. subsalsa*) (NIES-3373) and *Arthrospira platensis* (*A. platensis*) (NIES-39) (National Institute for Environmental Studies, Ibaraki Prefecture, Japan) (Fig. 5). *C. littorale* and *Stichococcus sp.* were cultured with a mixture of Daigo IMK medium (Nihon Pharmaceutical Co., Ltd., Tokyo, Japan) and Daigo artificial seawater SP (FujiFilm-Wako Pure Chemical Industries). *C. vulgaris, S. subsalsa*, *E. gracilis* and *A. platensis* were cultured with C medium, MA medium, AF-6 medium and SOT medium (National Institute for Environmental Studies, Ibaraki Prefecture, Japan), respectively. The microalgae were cultured using a suspension culture flask (AGC Techno Glass Co., Ltd., Shizuoka Prefecture, Japan) at room temperature (25°C), under continuous lighting (about 500 to 700 lux) [Reference 1]. The phase contrast images of the microalgae were taken with a microscope (ELIPSE TS2, Nikon Corp., Tokyo, Japan) and analyzed using software (NIS-Elements BR, Nikon Corp.).

### 1-2. Nutrient extraction from microalgae

Hydrolysis was used to extract the nutrients from the microalgae. The microalgae cultured in the flask were collected in a screw bottle (As One Corp., Osaka Prefecture, Japan) regardless of the culturing period, and dried in an oven (Tietech Co., Ltd., Saitama Prefecture, Japan). After drying, the dry weight was measured and purified water was added for adjustment to the optimal concentration (10 g/L or 50 g/L). Acid/alkali hydrolysis reaction was then conducted. Heating was carried out using an autoclave (Tomy Seiko Co., Ltd., Tokyo, Japan), a heat block (Labnet International Inc., New Jersey, U.S.) or a dry air sterilizer (Advantech, Inc., Tokyo, Japan). The heating conditions were as follows: Temperature: 20 to 180°C; heating time: 10 to 1440 min; sulfuric acid concentration: 0.0036 to 1.8 N; hydrochloric acid and sodium hydroxide concentration: 0.36 to 1 N. The experiment was conducted with different combinations of temperature, time and reagent concentration. After heat treatment, the mixture was neutralized with hydrochloric acid or sodium hydroxide. The glucose and amino acid concentrations were measured by quantitative analysis using the hexokinase method [Reference 2] and the liquid chromatography-mass spectrometry method.

### 1-3. Cell culturing and cell viability assay

C2C12 mouse myoblasts (Sumitomo-Dainippon Pharmaceutical Co., Ltd., Tokyo, Japan) were cultured with Dulbecco's Modified Eagle Medium (DMEM) (Sigma-Aldrich, Missouri, U.S.) supplemented with 10% fetal bovine serum (FBS, Thermo-Fischer Scientific, Massachusetts, U.S.) and 1% penicillin-streptomycin (P/S, Invitrogen, Carlsbad, California, U.S.), at 37°C under a moist atmosphere containing 5% CO₂ [Reference 3]. The cell proliferation and survival rates were evaluated by XTT assay (Biological Industries, Connecticut, U.S.). The C2C12 cells were inoculated into a 96-well plate (AGC Techno Glass Co., Ltd., Shizuoka Prefecture, Japan) at a density of 10,000 cells/well, and cultured overnight in DMEM supplemented with 10% FBS and 1% P/S. After overnight culturing, the medium was discarded and washing was performed twice with phosphate buffered saline (PBS, Sigma-Aldrich). Nutrient-deficient medium containing or not containing algal extract or glucose/glutamic acid was then used for culturing of the cells. The solution-added cells were then incubated for 2 days. In order to eliminate the difference in absorbance by the pigment in each solution, each solution was used with addition of 100 µL of 10% FBS and 1% P/S and 50 µL of XTT reagent, just before starting the XTT assay. The subsequent cell viability assay was carried out according to protocol. Glucose-free DMEM (Invitrogen), glucose/glutamine-free DMEM (Invitrogen) and glucose/amino acid-free DMEM (United States Biological, Massachusetts, U.S.) were used as nutrient-deficient media. Glucose (Kanto Kagaku Co., Ltd., Tokyo, Japan) or glutamic acid (FujiFilm-Wako Pure Chemical Industries) were added to each medium as necessary.

The media used in Examples 1 and 2 had the following compositions unless otherwise specified.
(A) Nutrient and mineral composition of DMEM used for ordinary animal cell culturing
   (i) Sugars
      - Glucose: 1000 mg/L or 4500 mg/L
      - Pyruvic acid: 110 mg/L
   (ii) Amino acids
      - L-arginine: 84 mg/L
      - L-cysteine: 48 mg/L
      - L-glutamine: 584 mg/L
      - Glycine: 30 mg/L
      - L-histidine: 42 mg/L
      - L-isoleucine: 105 mg/L
      - L-leucine: 105 mg/L
      - L-lysine: 146 mg/L
      - L-methionine: 30 mg/L
      - L-phenylalanine: 66 mg/L
      - L-serine: 42 mg/L
      - L-threonine: 95 mg/L
      - L-tryptophan: 16 mg/L
      - L-tyrosine: 71 mg/L
      - L-valine: 94 mg/L
   (iii) Vitamins
      - Pantothenic acid: 4 mg/L
      - Choline chloride: 4 mg/L
      - Folic acid: 4 mg/L
      - i-Inositol: 7.2 mg/L
      - Niacinamide: 4 mg/L
      - Pyridoxine: 4 mg/L
      - Riboflavin: 0.4 mg/L
      - Thiamine: 4 mg/L
   (iv) Minerals
      - CaCl₂: 200 mg/L
      - KCl: 400 mg/L
      - Fe(NO₃)₃ · 9H₂O: 0.10 mg/L
      - MgSO₄: 98 mg/L
      - NaCl: 6400 mg/L
      - NaHCO₃: 3700 mg/L
      - NaH₂PO₄: 109 mg/L
      - Phenol red: 15 mg/L
(B) Medium composition used in animal cell culture experiment with algal extract
   (1) DMEM (glucose-free)
      Algal extract was used to demonstrate its suitability as a substitute for sugars (glucose· pyruvic acid).
      (i) Sugars
         - Glucose: 0 mg/L
         - Pyruvic acid: 0 mg/L
      (ii) Amino acids
         - L-arginine: 84 mg/L
         - L-cysteine: 48 mg/L
         - L-glutamine: 584 mg/L
         - Glycine: 30 mg/L
         - L-histidine: 42 mg/L
         - L-isoleucine: 105 mg/L
         - L-leucine: 105 mg/L
         - L-lysine: 146 mg/L
         - L-methionine: 30 mg/L
         - L-phenylalanine: 66 mg/L
         - L-serine: 42 mg/L
         - L-threonine: 95 mg/L
         - L-tryptophan: 16 mg/L
         - L-tyrosine: 71 mg/L
         - L-valine: 94 mg/L
      (iii) Vitamins
         - Pantothenic acid: 4 mg/L
         - Choline chloride: 4 mg/L
         - Folic acid: 4 mg/L
         - i-Inositol: 7.2 mg/L
         - Niacinamide: 4 mg/L
         - Pyridoxine: 4 mg/L
         - Riboflavin: 0.4 mg/L
         - Thiamine: 4 mg/L
      (iv) Minerals
         - CaCl₂: 200 mg/L
         - KCl: 400 mg/L
         - Fe(NO₃)₃ · 9H₂O: 0.10 mg/L
         - MgSO₄: 98 mg/L
         - NaCl: 6400 mg/L
         - NaHCO₃: 3700 mg/L
         - NaH₂PO₄: 109 mg/L
         - Phenol red: 15 mg/L
   (2) DMEM (glucose/amino acid-free)
      Algal extract was used to demonstrate its suitability as a substitute for sugars (glucose· pyruvic acid) and amino acids (L-arginine, L-cysteine, L-glutamine, glycine, L-histidine, L-isoleucine, L-leucine, L-lysine, L-methionine, L-phenylalanine, L-serine, L-threonine, L-tryptophan, L-tyrosine, L-valine).
      (i) Sugars
         - Glucose: 0 mg/L
         - Pyruvic acid: 0 mg/L
      (ii) Amino acids
         - L-arginine: 0 mg/L
         - L-cysteine: 0 mg/L
         - L-glutamine: 0 mg/L
         - Glycine: 0 mg/L
         - L-histidine: 0 mg/L
         - L-isoleucine: 0 mg/L
         - L-leucine: 0 mg/L
         - L-lysine: 0 mg/L
         - L-methionine: 0 mg/L
         - L-phenylalanine: 0 mg/L
         - L-serine: 0 mg/L
         - L-threonine: 0 mg/L
         - L-tryptophan: 0 mg/L
         - L-tyrosine: 0 mg/L
         - L-valine: 0 mg/L
      (iii) Vitamins
         - Pantothenic acid: 4 mg/L
         - Choline chloride: 4 mg/L
         - Folic acid: 4 mg/L
         - i-Inositol: 7.2 mg/L
         - Niacinamide: 4 mg/L
         - Pyridoxine: 4 mg/L
         - Riboflavin: 0.4 mg/L
         - Thiamine: 4 mg/L
      (iv) Minerals
         - CaCl₂: 200 mg/L
         - KCl: 400 mg/L
         - Fe(NO₃)₃ · 9H₂O: 0.10 mg/L
         - MgSO₄: 98 mg/L
         - NaCl: 6400 mg/L
         - NaHCO₃: 3700 mg/L
         - NaH₂PO₄: 109mg/L
         - Phenol red: 15 mg/L
   (3) Inorganic salt medium
      Algal extract was used to demonstrate its suitability as a substitute for sugars (glucose· pyruvic acid), amino acids (L-arginine, L-cysteine, L-glutamine, glycine, L-histidine, L-isoleucine, L-leucine, L-lysine, L-methionine, L-phenylalanine, L-serine, L-threonine, L-tryptophan, L-tyrosine, L-valine) and vitamins (pantothenic acid, choline chloride, folic acid, i-inositol, niacinamide, pyridoxine, riboflavin and thiamine).
      (i) Sugars
         - Glucose: 0 mg/L
      (ii) Amino acids
         - L-arginine: 0 mg/L
         - L-cysteine: 0 mg/L
         - L-glutamine: 0 mg/L
         - Glycine: 0 mg/L
         - L-histidine: 0 mg/L
         - L-isoleucine: 0 mg/L
         - L-leucine: 0 mg/L
         - L-lysine: 0 mg/L
         - L-methionine: 0 mg/L
         - L-phenylalanine: 0 mg/L
         - L-serine: 0 mg/L
         - L-threonine: 0 mg/L
         - L-tryptophan: 0 mg/L
         - L-tyrosine: 0 mg/L
         - L-valine: 0 mg/L
      (iii) Vitamins
         - Pantothenic acid: 0 mg/L
         - Choline chloride: 0 mg/L
         - Folic acid: 0 mg/L
         - i-Inositol: 0 mg/L
         - Niacinamide: 0 mg/L
         - Pyridoxine: 0 mg/L
         - Riboflavin: 0 mg/L
         - Thiamine: 0 mg/L
      (iv) Minerals
         - CaCl₂: 200 mg/L
         - KCl: 400 mg/L
         - Fe(NO₃)₃ · 9H₂O: 0.10 mg/L
         - MgSO₄: 98 mg/L
         - NaCl: 6400 mg/L
         - NaHCO₃: 3700 mg/L
         - NaH₂PO₄: 109 mg/L
         - Phenol red: 15 mg/L

### 1-4. Data analysis

All of the data in the drawings are represented as mean ±SD. Unpaired Student t-test was carried out for comparison of two groups, while one-way ANOVA with post-hoc Tukey's HSD test was used for comparison of multiple groups.

### 2. Results

### 2-1. Glucose extraction from microalgae.

Initially, it was attempted to extract glucose, as an important nutrient for mammalian cell medium. In a preliminary experiment, glucose extraction efficiency by acid hydrolysis using sulfuric acid was highest with Chlorococcum (C. *littorale*) among the six microalgae shown in Fig. 5. The optimal conditions were therefore examined using C. *littorale.* Glucose was efficiently extracted from microalgae by high-temperature treatment under pressure using an autoclave (sulfuric acid concentration: 0.18 to 0.36 N; temperature: 130 to 135°C; reaction time: 10 to 20 min) (Fig. 1A). The glucose yield with heat treatment (130°C) but without pressurizing with a dry air sterilizer was lower than by autoclave treatment at the same temperature (Fig. 1A). These results suggest that pressure is also important in addition to temperature for efficient glucose extraction.

It was then examined which microalgae from among the six microalgae C. *littorale, Stichococcus* sp., *S. subsalsa*, *C. vulgaris*, *E. gracilis* and *A. platensis* had the highest glucose yield (Fig. 5). While glucose was recoverable from all of the tested microalgae, glucose was extracted more efficiently from C. *littorale* and *A. platensis* (Fig. 1B).

In order to obtain a microalgal extract with a high glucose concentration, the initial algae concentration was increased from 10 g/L to 50 g/L. The concentration of glucose extracted from *C. littorale* was 2.4 ± 0.3 g/L with 10 g/L, and 13.1 ± 1.5 g/L with 50 g/L. The concentration of glucose extracted from *A. platensis* was 1.9 ± 0.3 g/L with 10 g/L, and 10.8 ± 0.6 g/L with 50 g/L (Fig. 1C). These results suggested that the glucose concentration had also increased proportional to the microalgae concentration. The algal extracts contained glucose in amounts of 3 to 4 times that of conventional mammal culture medium (DMEM supplemented with 10% FBS and 1% P/S) (Fig. 1C).

### 2-2. Amino acid extraction from microalgae.

DMEM contains 15 of the 20 different proteinogenic amino acids. The 15 amino acids are the amino acids arginine to valine, shown at left in Fig. 2. Quantitative analysis of the amino acids in the C. *littorale* extract was carried out with 0.36 N sulfuric acid for 20 minutes at 130°C. The concentrations of all of the 15 amino acids in the algal extract were lower than in the medium (Fig. 2A). Heat treatment is conventionally carried out for 24 hours for efficient acid hydrolysis of proteins [Reference 4]. To increase the extraction efficiency, therefore, 1 N sulfuric acid was used for extraction of the amino acids at 100°C for 24 hours. The concentration of extracted amino acids was increased by this method (Fig. 2A). Next, 1 N hydrochloric acid or 1 N sodium hydroxide was used in an attempt to further increase the extraction efficiency. The analysis results indicated that all of the amino acids extracted with hydrochloric acid were higher than the amino acids extracted with sulfuric acid (Fig. 2A). However, glutamine and tryptophan that are included in DMEM were not detected under any of the conditions (Fig. 2A). Since glutamine was hydrolyzed to glutamic acid during the acid hydrolysis, glutamine could not be detected in the extract, and a high-concentration of glutamic acid was detected instead. Although amino acids were efficiently detected even when using sodium hydroxide, the 3 amino acids in DMEM (arginine, cysteine and histidine) were detected only by acid hydrolysis. Acid hydrolysis using 1 N hydrochloric acid at 100°C for 24 hours was therefore used for extraction of amino acids.

It was then examined which among the 6 microalgae had the maximum amino acid yield (Fig. 5). It was found that among these microalgae, the extract from C. *vulgaris* had the highest abundance of amino acids (Fig. 2B). These results coincided with the relatively high protein concentration of C. *vulgaris* found in previous research [References 5 and 6]. It was demonstrated that, in addition to the amino acids decomposed by hydrolysis, most of the amino acids in any of the algae species were recovered in concentrations that were the same or higher than in mammalian cell medium (Fig. 2B).

In order to obtain a microalgal extract with a higher amino acid concentration, the initial algae concentration was increased from 10 g/L to 50 g/L. Similar to extraction of glucose, the concentration of each amino acid was likewise further increased by increasing the algae concentration (Fig. 2C).

No glutamine was extracted by acid hydrolysis, but glutamine was abundantly present in the DMEM (Fig. 2). Glutamic acid, on the other hand, was present in the microalgal extract but the DMEM contained no glutamic acid (Fig. 2). The very trace amount of glutamic acid detected in Fig. 2 is thought to have been either from the supplemented 10% FBS or produced by glutamine decomposition. It was therefore examined whether or not glutamic acid can be used as a surrogate for glutamine in mammalian cell culturing.

Most of the C2C12 mouse myoblasts had died after 2 days of culturing with glucose- and glutamine-free medium (Fig. 3). Addition of glucose partly increased the cell survival rate, which was still further increased with addition of glutamic acid (Fig. 3). This suggested that glutamic acid was utilized as a surrogate for glutamine during culturing of the mammalian cells. However, the increase by addition of glutamic acid was less than with glutamine-containing medium (Fig. 3). It was therefore conjectured that excess addition of glutamic acid is necessary.

### 2-3. Cell culturing with microalgal extract

It was next examined whether or not algal extract can be utilized as a nutrient in mammalian cell culturing. Most of the C2C12 mouse myoblasts had died after 2 days of culturing with glucose-free medium (Fig. 4A). However, addition of C. *littorale* extract at 5%, 10% and 20% restored the cell survival rate (Fig. 4A), reaching a plateau at 10% addition. The results verified that algal extract is used as a surrogate for glucose which is an important nutrient for culturing of mammalian cell.

Finally, it was examined whether or not algal extract can be utilized as a surrogate for amino acids. Glucose- and amino acid-free medium was used for this experiment. Most of the cells died after culturing for 2 days using this medium (Figs. 4B, 4C and 4D). However, the cell survival rate was restored by addition of 5% and 10% microalgal extract (Figs. 4B, 4C and 4D). When C. *littorale* extract was added at 5%, 10% and 20%, the cell survival rate tended to be higher than without addition (Fig. 4B). With *A. platensis* and C. *vulgaris,* the cell survival rate did not recover even with excess addition (20%) of extract (Figs. 4C and 4D).

Cells were cultured using combinations of glucose-rich C. *littorale* and amino acid-rich C. *vulgaris* and glucose-rich *A. platensis* and amino acid-rich C. *vulgaris* extracts (Fig. 4E). The extract combinations both produced higher survival rates than without addition of extract (Fig. 4E). The results verified that algal extract can be used as a surrogate for amino acids that are important nutrients for cell culturing.

The results verified that nutrients extracted from microalgae can be utilized as surrogates for glucose and amino acids for mammalian cells.

The results described above are only for analysis of glucose and amino acids. Lipids are not included in DMEM. Eight different vitamin B molecules are present in DMEM, but at much lower concentrations than glucose and amino acids. Experimentation by the present inventors has shown that the 8 different vitamin B molecules can also be extracted from microalgae. Therefore, it is believed that all of the nutrients and vitamins necessary for mammalian cell culturing can be provided from microalgae.

Algal extracts were shown to function as a surrogates for nutrients during mammalian cell culturing. Since the use of cereals for production of biofuels is in competition with food, it is a promising alternative to use microalgae which are not in competition with food [Reference 7]. The present research has demonstrated the possibility not only of novel cell culturing systems which can reduce effects on the environment and protect against environmental change, but also of the alternative of using microalgae instead of cereal-dependent culture systems.

### <Example 2>

Liquid extract was prepared from *Chlorella vulgaris* Beijerinck by the method of Example 1, 1-2. The medium used was DMEM or inorganic salt medium, and the effect of *Chlorella* algal extract on culturing of cow muscle tissue cells was investigated. The experiment was carried out by the method described in Example 1, 1-3. The cow muscle tissue cells were cow cheek muscle cells purchased from Tokyo Meat Market Co., Ltd. and treated with protease, and the cells were isolated for use.

The results demonstrated that addition of algal extract to inorganic salt medium allowed maintenance culturing of cow muscle tissue cells similar to DMEM (Fig. 6).

### <Example 3>

Liquid extract was prepared from *Chlorella vulgaris* Beijerinck by the method of Example 1, 1-2. The medium used was DMEM or inorganic salt medium, and the effect of the presence or absence of *Chlorella* algal extract and the presence or absence of fetal bovine serum (FBS) on culturing of cow muscle tissue cells was investigated. The experiment was carried out by the method described in Example 1, 1-3.

As a result, a tendency toward increased relative viable cell count of cow muscle tissue cells was found with addition of algal extract to inorganic salt medium, and with further addition of serum (Fig. 7).

### <Example 4>

Liquid extract was prepared from *Chlorella vulgaris* Beijerinck by the method of Example 1, 1-2. The medium used was DMEM or inorganic salt medium, and the effect of the presence or absence of *Chlorella* algal extract and the presence or absence of fetal bovine serum (FBS) on culturing of cow muscle tissue cells, especially during the cell growth stage, was investigated. The experiment was carried out by the method described in Example 1, 1-3.

As a result, growth of cow muscle tissue cells was found with addition of algal extract to inorganic salt medium, and with further addition of serum, on the same level as with 10% FBS DMEM (Fig. 8).

### References:

[1] Y. Haraguchi, Y. Kagawa, K. Sakaguchi, K. Matsuura, T. Shimizu, et al., Thicker three-dimensional tissue from a "symbiotic recycling system" combining mammalian cells and algae. Sci. Rep. 7 (2017) 41594.
[2] Y. Haraguchi, W. Sekine, T. Shimizu, M. Yamato, S. Miyoshi, A. et al., Development of a new assay system for evaluating the permeability of various substances through three-dimensional tissue. Tissue Eng. Part C Methods. 16 (2010) 685-692.
[3] Y. Haraguchi, T. Shimizu, T. Sasagawa, H. Sekine, K. Sakaguchi, et al., Fabrication of functional three-dimensional tissues by stacking cell sheets in vitro. Nat. Protoc. 7 (2012) 850-858.
[4] S. McCusker, P.R. Buff, Z. Yu, A.J Fascetti, Amino acid content of selected plant, algae and insect species: a search for alternative protein sources for use in pet foods. J. Nutr. Sci. 3 (2014)e39.
[5] E.W. Becker, Micro-algae as a source of protein. Biotechnol. Adv. 25 (2007) 207-210.
[6] P. Spolaore, C. Joannis-Cassan, E. Duran, A. Isambert, Commercial applications of microalgae. J. Biosci. Bioeng. 101 (2006) 87-96.
[7] S.H. Ho, S.W. Huang, C.Y. Chen, T. Hasunuma, A. Kondo, et al., Bioethanol production using carbohydrate-rich microalgae biomass as feedstock. Bioresour. Technol. 135 (2013) 191-198.

## Claims

1. A production method for a composition for cell culturing, wherein the method comprises the following steps:
(1) subjecting algae to acid hydrolysis and/or alkali hydrolysis;
(2) neutralizing the hydrolysate obtained from step (1) to obtain an algal extract; and
(3) mixing the algal extract with cell culturing medium, wherein the cell culturing medium contains substantially no L-glutamine.

2. The method according to claim 1, wherein the cell culturing medium is also substantially free of one or more amino acids selected from the group consisting of L-arginine, L-cysteine, glycine, L-histidine, L-isoleucine, L-leucine, L-lysine, L-methionine, L-phenylalanine, L-serine, L-threonine, L-tryptophan, L-tyrosine and L-valine.

3. The method according to claim 1 or 2 wherein the cell culturing medium contains substantially no vitamins.

4. The method according to any one of claims 1 to 3, wherein the cell culturing medium contains substantially no glucose.

5. The method according to any one of claims 1 to 4, wherein the cell culturing medium is an inorganic salt medium.

6. The method according to any one of claims 1 to 5, wherein the cultured cells are animal cells.

7. The method according to any one of claims 1 to 6, wherein the algae are unicellular algae.

8. The method according to any one of claims 1 to 7, wherein the algae are at least one type selected from the group consisting of unicellular green algae, unicellular blue-green algae, unicellular red algae, unicellular charales and unicellular ulvophytes.

9. The composition according to any one of claims 1 to 8, wherein the algae are at least one type selected from the group consisting of *Chlorococcum littorale*, *Stichococcus sp*., *Chlorella vulgaris* Beijerinck, *Euglena gracilis, Spirulina subsalsa* and *Arthrospira platensis.*

10. The method according to any one of claims 1 to 9, wherein step (1) is carried out under pressure.

11. The method according to any one of claims 1 to 10, wherein step (1) is carried out at 60°C to 200°C.

12. A composition for cell culturing which is obtained by the method according to any one of claims 1 to 11.

13. A cell culturing method using a composition for cell culturing which is obtained by the method according to any one of claims 1 to 11.
